# EUROPEAN PATENT SPECIFICATION

(11) **EP 4 217 508 B1**
(45) Date of publication and mention of the grant of the patent: **28.01.2026**
(21) Application number: 21786530.2
(22) Date of filing: 15.09.2021
(51) Int. Cl.: C12Q 1/6883

(54) **APPARATUS, KITS AND METHODS FOR PREDICTING THE DEVELOPMENT OF SEPSIS**
VORRICHTUNG, KITS UND VERFAHREN ZUR VORHERSAGE DER ENTWICKLUNG VON SEPSIS
APPAREILS, KITS ET PROCÉDÉS POUR PRÉDIRE LE DÉVELOPPEMENT D'UNE SEPTICÉMIE

(30) Priority: 22.09.2020 GR 20200100575
(43) Date of publication of application: 02.08.2023
(73) Proprietor: THE SECRETARY OF STATE FOR DEFENCE, Salisbury, Wiltshire SP4 0JQ (GB)
(72) Inventor: LUKASZEWSKI, Roman Antoni, Salisbury, Wiltshire SP4 0JQ (GB); PANAGIOTOU, Ioannis, 10405 Berlin (DE); SCHMIDT-HECK, Wolfgang, 07749 Jena (DE)
(74) Representative: Phillips, Thomas Edward
(86) International application number: PCT/GB2021/000102
(87) International publication number: WO 2022/064163

(56) References cited:
- WO-A1-2015/121605
- WO-A1-2017/106918
- WO-A2-2006/113529
- GAVIN C. K. W. KOH ET AL: "Host Responses to Melioidosis and Tuberculosis Are Both Dominated by Interferon-Mediated Signaling", PLOS ONE, vol. 8, no. 1, 1 January 2013 (2013-01-01), pages e54961, XP055376146, DOI: 10.1371/journal.pone.0054961
- ILLUMINA: "HumanHT-12 v4 BeadChip Product Information", 1 January 2010 (2010-01-01), pages 1 - 2, XP055178072, Retrieved from the Internet <URL:http://www.illumina.com/documents/products/product_information_sheets/product_info_humanht-12.pdf> [retrieved on 20150320]
- AMBION: "Illumina TotalPrep TM RNA Amplification Kit", 1 January 2011 (2011-01-01), pages 1 - 30, XP055178075, Retrieved from the Internet <URL:http://tools.lifetechnologies.com/content/sfs/manuals/IL1791ME.pdf> [retrieved on 20150320]

## Description

The present invention is concerned with kits, methods and apparatus for analysing a biological sample from a subject to predict and/or monitor the development of infection and/or organ dysfunction and/or sepsis utilising groups of nucleic acid markers to predict the development of infection and/or organ dysfunction and/or sepsis. The invention is defined in the appended claims.

Following exposure to a microbial pathogen there is often a lag phase before symptoms of infection, which could further result in symptoms of organ dysfunction, and development of sepsis. After the onset of clinical symptoms, the effectiveness of treatment often decreases as the disease progresses, so the time taken to make any diagnosis is critical. It is likely that a detection or diagnostic assay will be the first confirmed indicator of infection, organ dysfunction or sepsis. The availability, rapidity and predictive accuracy of such an assay will therefore be crucial in determining the outcome. Any time saved will speed up the implementation of medical countermeasures and will have a significant impact on recovery.

The development of technologies to facilitate rapid detection of infection, organ dysfunction and sepsis is a key concern for all at risk. During the initial stages of infection many biological agents are either absent from, or present at very low concentrations in, typical clinical samples (e.g. blood). It is therefore likely that agent-specific assays would have limited utility in detecting infection before clinical symptoms arise. Previous studies have shown that infection elicits a pattern of immune response involving changes in the expression of a variety of biomarkers that is indicative of the type of agent. Such patterns of biomarker expression have proven to be diagnostic for a variety of infectious agents. It is now possible to distinguish patterns of gene expression in blood leukocytes from symptomatic patients with acute infections caused by four common human pathogens (Influenza A, *Staphylococcus aureus, Streptococcus pneumoniae* and *Escherichia coli*) using whole transcriptome analysis. More recently, researchers have been able to reduce the number of host biomarkers required to make a diagnosis through use of appropriate bioinformatic analysis techniques to select key biomarkers for the diagnosis of infectious disease.

While host biomarker signatures represent an attractive solution for the prediction of microbial infection, their discovery relies on the exploitation of laboratory models of infection whose fidelity to the pathogenesis of disease in humans varies. An alternative approach for biomarker discovery in humans is to exploit a common sequela of biological agent infection; such as the life-threatening condition sepsis, which now requires organ dysfunction for a positive diagnosis. Sepsis has traditionally been defined as a systemic inflammatory response syndrome (SIRS) in response to infection which, when associated with acute organ dysfunction, may ultimately cause severe life-threatening complications. However, sepsis is now defined as life-threatening organ dysfunction caused by a dysregulated host response to infection, wherein organ dysfunction can be identified as an increase in the total sequential organ failure assessment (SOFA) score of ≥2 or more points from one day to the next following infection. Severity of organ dysfunction has in the past been assessed with various scoring systems that quantify abnormalities according to clinical findings, laboratory data, or therapeutic interventions. The predominant score in current use is the SOFA (originally the Sepsis-related Organ Failure Assessment). A higher SOFA score is associated with an increased probability of mortality. The score grades abnormality by organ system and accounts for clinical interventions. The baseline SOFA score can be assumed to be zero in patients not known to have pre-existing organ dysfunction. A SOFA score ≥2 reflects an overall mortality risk of approximately 10% in a general hospital population with suspected infection.

Sepsis is a major cause of morbidity and mortality in intensive care units (ICU). In the UK, sepsis is believed to be responsible for about 27% of all ICU admissions. Across Europe the average incidence of sepsis in the ICU is about 30%, with a mortality rate of 27%. In the USA, hospital-associated mortality from sepsis ranges between 18 to 30%; an estimated 9.3% of all deaths occurred in patients with sepsis. Clearly there is a very accessible patient population that could be used to study predictive markers for the onset of sepsis.

Despite greatly improved diagnosis, treatment and support, serious infection and sepsis remain significant causes of death and often result in chronic ill-health or disability in those who survive acute episodes. Although sudden, overwhelming infection is comparatively rare amongst otherwise healthy adults, it constitutes an increased risk in immunocompromised individuals, seriously ill patients in intensive care, burns patients and young children. In a proportion of cases, an apparently treatable infection leads to the development of sepsis; a dysregulated, inappropriate response to infection characterised by progressive circulatory collapse leading to renal and respiratory failure, abnormalities in coagulation, profound and unresponsive hypotension and, in about 30% of cases death. The incidence of sepsis in the population of North America is about 0.3% of the population annually (about 750,000 cases) with mortality rising to 40% in the elderly and to 50% in cases of the most severe form, septic shock.

The ability to detect potentially serious infections and organ dysfunction as early as possible and, especially, to predict the onset of sepsis in susceptible individuals is clearly advantageous.

Although a number of biomarkers (markers), such as nucleic acid markers or protein markers, have been shown to correlate with sepsis and some give an indication of the seriousness of the condition, no single marker or combination of markers has yet been shown to be a reliable diagnostic test, much less a predictor of the development of sepsis, especially sepsis meeting the criteria of the new definition, requiring life-threatening organ dysfunction.

Extracting reliable diagnostic patterns and robust prognostic indications from changes over time in complex sets of variables including traditional clinical observations, clinical chemistry, biochemical, immunological and cytometric data requires sophisticated methods of analysis. The use of expert systems and artificial intelligence, including neural networks, for medical diagnostic applications has been being developed for some time.

The ability to detect the earliest signs of infection and/or organ dysfunction and/or sepsis has clear benefits in terms of allowing treatment as soon as possible. Indications of the severity of the condition and likely outcome if untreated inform decisions about treatment options. This is relevant both in vulnerable hospital populations, such as those in intensive care, or who are burned or immunocompromised, and in other groups in which there is an increased risk of serious infection and subsequent sepsis. The use or suspected use of biological weapons in both battlefield and civilian settings is an example where a rapid and reliable means of testing for the earliest signs of infection or organ dysfunction (i.e. sepsis) in individuals exposed would also be advantageous. WO 2015/121605 A1 (2015-08-20) is concerned with kits, methods and apparatus for analysing a biological sample from an animal to predict and monitor the development of sepsis utilising biomarker signatures/lists of biomarkers, inter alia TDRD9 to predict whether an animal is likely to develop the symptoms of sepsis.

WO 2017/106918 A1 (2017-06-29) provides methods, apparatus, kits and compositions for determining the absence of a systemic bacterial infection (sepsis) in patients. The expression level of different genes, inter alia, PAFAH2 and SLC39A8, is determined.

However, until now the majority of investigations focussed on developing a group of biomarkers and/or a test for sepsis were based on the previous definition of sepsis of systemic inflammatory response syndrome (SIRS) in response to infection, and thus have generally focussed on identifying a group of biomarkers and/or a test to predict SIRS in response to infection. Sepsis is however now defined as life-threatening organ dysfunction caused by a dysregulated host response to infection, and thus a group of biomarkers and/or a test is required which is capable of identifying individuals at risk of developing life-threatening organ dysfunction caused by a dysregulated host response to infection. Until now neither a test nor a list of biomarkers has been identified/produced which can detect or predict sepsis (organ dysfunction) with a good/high predictive accuracy (for example with an area under the curve (AUC) > 0.8, but preferably > 0.85, and most preferably > 0.9).

The present invention thus aims to provide biomarker signatures (groups of biomarkers), and methods for classifying biological samples using the biomarker signatures, to predict/detect the development of organ dysfunction, and consequently sepsis, and optionally both infection and organ dysfunction, and sepsis, with a high predictive accuracy.

With this in mind, the applicants have determined several lists/groups (signatures) of nucleic acid markers (biomarkers) which can be used to predict the development of infection and/or organ dysfunction, and thus predict the development of sepsis, with the possibility of predicting prior to the onset of symptoms (pre-symptomatic). The Applicant has identified through a comprehensive analysis of the host transcriptome, sourced from blood samples from human patients collected prior to the clinical onset of sepsis, a panel of 157 nucleic acids highly significant to predicting infection and organ dysfunction, and subsets thereof for predicting development of infection and/or organ dysfunction and/or sepsis.

The 157 nucleic acid markers highly significant to predicting infection and organ dysfunction are:
AC005747.1, AC112777.1, AC132942.1, AC141557.1, ACVR1B, ADCK2, AFDN, AL096803.2, AL133444.1, ANKS1A, ARHGEF40, ARL4A, ASB1, ASPM, ATP5MD, B4GALT5, BAG3, BEX1, BTNL8, C1orf116, C1orf226, C22orf46, C7orf50, CACTIN, CBS, CCDC32, CCNY, CD177P1, CD58, CDCA7, CDH23, CENPE, CGTLC4P, CLASP1, CLECL1, CLN3, CPA3, CPEB4, CXCL16, CXCR2P1, DHRS9, DLGAP5, EIF1AY, EIF4G3, FAM83A, FAR2, FBN2, FBXL18, FBXW9, FCER1A, FCMR, FHDC1, FOCAD, FOXI3, GAS6, GAS7, GGTLC4P, GIMAP5, GRAP2, H2BC11, HBD, HCAR2, HCAR3, HIPK2, HLA-DMB, HNMT, HROB, ICAM2, ID3, IL18RAP, INKA1, IRF5, IRX3, JUP, KCNC4, KIR2DL4, KMTSB, LARP1, LDLR, LDLRAP1, LINC01347, LINC02363, LMNA, LRRN3, LTA, MAD1L1, MBOAT2, MIAT, MRPS6, MSANTD1, MTRR, N4BP2, NABP1, NCAPG2, NCOR2, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SLC51A, SMG1, SNAPC2, SNHG5, SNRPN, SPC24, SPTLC2, SQLE, SRRM2, STAB1, TACSTD2, TCAF1, TDRD9, TEX2, THBS3, THEM6, TK1, TLE1, TMEM116, TMEM144, TMEM183A, TMTC1, TOP2A, TSC22D1, TTC39C, UGP2, UVSSA, VNN1, VPS9D1, ZBTB16, ZNF608, ZNF792.

Details of the particular nucleic acids, from their annotated names (as provided throughout this application), can be found from human gene databases such as GeneCards^{®}: The Human Gene Database (genecards.org), or the Human Gene Resources at the National Center for Biotechnology Information (NCBI) (ncbi.nlm.nih.gov/genome/guide/human/).

Organ dysfunction and infection are two key indicators of sepsis, and thus there is a need to identify markers which are capable of predicting development of either, though at least organ dysfunction, but preferably both, with good levels of predictivity, and as early as possible, and preferably before symptoms occur. There is especially however a need to identify groups of markers (signatures) which can specifically predict development of organ dysfunction, and with high levels of predicitvity, and this is a key requirement for sepsis. The Applicant has down-selected lists of nucleic acid markers critical to predicting sepsis with a high level of confidence in the prediction, which are capable of providing a prediction up to at least three days in advance of symptoms.

Subsets of the group of 157 nucleic acid markers, in particular subsets from as few as 4 biomarkers, have been shown to be capable of predicting development of infection versus absence of infection, predicting development of organ dysfunction versus non-complicated infection, and predicting organ dysfunction versus absence of organ dysfunction, thereby predicting sepsis versus absence of sepsis, through analysis of samples from subjects (for example patients) categorised as progressing to development of sepsis, or not, or as progressing to having an infection, or not.

Thus in a first aspect the present description, which is not part of the invention, provides a method for predicting the development of infection and/or organ dysfunction and/or sepsis in a subject, the method comprising determining levels of at least four nucleic acid markers, or a product expressed by those nucleic acids, such as the corresponding proteins, in a biological sample taken from the subject, wherein the at least four nucleic acid markers are selected from the list consisting of:
AC005747.1, AC112777.1, AC132942.1, AC141557.1, ACVR1B, ADCK2, AFDN, AL096803.2, AL133444.1, ANKS1A, ARHGEF40, ARL4A, ASB1, ASPM, ATP5MD, B4GALT5, BAG3, BEX1, BTNL8, C1orf116, C1orf226, C22orf46, C7orf50, CACTIN, CBS, CCDC32, CCNY, CD177P1, CD58, CDCA7, CDH23, CENPE, CGTLC4P, CLASP1, CLECL1, CLN3, CPA3, CPEB4, CXCL16, CXCR2P1, DHRS9, DLGAP5, EIF1AY, EIF4G3, FAM83A, FAR2, FBN2, FBXL18, FBXW9, FCER1A, FCMR, FHDC1, FOCAD, FOXI3, GAS6, GAS7, GGTLC4P, GIMAP5, GRAP2, H2BC11, HBD, HCAR2, HCAR3, HIPK2, HLA-DMB, HNMT, HROB, ICAM2, ID3, IL18RAP, INKA1, IRF5, IRX3, JUP, KCNC4, KIR2DL4, KMTSB, LARP1, LDLR, LDLRAP1, LINC01347, LINC02363, LMNA, LRRN3, LTA, MAD1L1, MBOAT2, MIAT, MRPS6, MSANTD1, MTRR, N4BP2, NABP1, NCAPG2, NCOR2, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SLC51A, SMG1, SNAPC2, SNHG5, SNRPN, SPC24, SPTLC2, SQLE, SRRM2, STAB1, TACSTD2, TCAF1, TDRD9, TEX2, THBS3, THEM6, TK1, TLE1, TMEM116, TMEM144, TMEM183A, TMTC1, TOP2A, TSC22D1, TTC39C, UGP2, UVSSA, VNN1, VPS9D1, ZBTB16, ZNF608, ZNF792,
wherein the levels of the at least four nucleic acid markers, or the products expressed by those nucleic acids, are used to predict the development of infection and/or organ dysfunction and/or sepsis.

The levels of the nucleic acid markers, or the products expressed by those nucleic acids, are preferably collectively, and in combination, used to predict the development of infection and/or organ dysfunction and/or sepsis, which could be through use of a mathematical model applied to the levels of the nucleic acid markers, or the products expressed by those nucleic acids, to provide the prediction.

The method of the first aspect may comprise use of a control in the method from which to establish the level (or expression level) of each nucleic acid, or a product thereof. The control may for example be one or more housekeeping genes/nucleic acids whose expression is predictable or relatively static irrespective of whether infection and/or organ dysfunction may develop. For example, the reference gene/nucleic acid may be FAM105B and/or RANBP3.

The term 'biological sample' includes, but not exclusively, blood, serum, plasma, urine, saliva, cerebrospinal fluid or any other form of material, preferably fluid-based or capable of being converted into a fluid-like state (e.g. tissue which can be broken down or separated in a solution, such as a buffered solution), which can be extracted or collected from a patient.

For prediction of infection versus absence of infection the Applicant has in particular identified 30 markers from the list of 157 nucleic acid markers which are significant to a prediction, with selections preferably of at least 7 or at least 8 markers from the 30 being particularly effective for providing a prediction of infection versus an absence of infection, with a high level of predictivity.

The group of 30 markers is: AC005747.1, ACVR1B, ANKS1A, ARHGEF40, B4GALT5, CBS, CD177P1, CDH23, CGTLC4P, EIF4G3, FBXL18, FOXI3, GAS7, GGTLC4P, HIPK2, IRX3, LDLR, LMNA MIAT, MTRR, NCAPG2, NCOR2, OPLAH, SLC51A, SMG1, SPTLC2, SQLE, THBS3, TLE1, TOP2A, and VPS9D1.

For example, the following subsets from the list of 30 markers are capable of achieving an AUC > 0.9 for predicting development of infection on specific days prior to development of symptoms:
1. SPTLC2, LDLR, EIF4G3, FBXL18, GGTLC4P, ARHGEF40, CD177P1, CDH23 (Day-1 before symptoms)
2. SLC51A, B4GALT5, THBS3, MTRR, IRX3, CBS, TOP2A, NCAPG2 (Day-2 before symptoms)
3. CGTLC4P, GAS7, NCOR2, FOXI3, TLE1, SQLE, SMG1, LMNA (Day-3 before symptoms)

These subsets of markers may be optimal for a specific day before the onset of symptoms of infection, or sepsis, although may be used in a test to identify development of infection or sepsis at any point before or after onset of symptoms, whereas other subsets of markers from the list of 30 markers may also be used for providing predictions, on individual days before symptoms or multiple days before symptoms, for example other subsets of markers from the list of 30 may be capable of predicting development of infection (or sepsis) with an AUC > 0.9 irrespective of the number of days prior to onset of symptoms, up to at least three days prior to onset of symptoms. For example, the 12 marker subset of ACVR1B, ANKS1A, B4GALT5, VPS9D1, EIF4G3, FBXL18, AC005747.1, HIPK2, LDLR, MIAT, OPLAH, SPTLC2.

Key nucleic acid markers, present in multiple (2 or more) down-selected subsets for prediction of infection versus no-infection are B4GALT5, EIF4G3, FBXL18, GGTLC4P, SPTLC2.

The selection of markers thus may comprise one or more of the key nucleic acid markers that recur during down-selection of subsets of markers.

For prediction of organ dysfunction versus absence of organ dysfunction the Applicant has in particular identified 131 markers which are significant to a prediction, with selections of at least 4, though optimally at least 7 or at least 8, from the 131 being particularly effective for providing a prediction of organ dysfunction versus absence of organ dysfunction with a high level of predictivity.

The group of 131 markers is: AC112777.1, AC132942.1, AC141557.1, ADCK2, AFDN, AL096803.2, AL133444.1, ANKS1A, ARL4A, ASB1, ASPM, ATP5MD, B4GALT5, BAG3, BEX1, BTNL8, C1orf116, C1orf226, C22orf46, C7orf50, CACTIN, CCDC32, CCNY, CD58, CDCA7, CENPE, CLASP1, CLECL1, CLN3, CPA3, CPEB4, CXCL16, CXCR2P1, DHRS9, DLGAP5, EIF1AY, FAM83A, FAR2, FBN2, FBXW9, FCER1A, FCMR, FHDC1, FOCAD, GAS6, GIMAP5, GRAP2, H2BC11, HBD, HCAR2, HCAR3, HLA-DMB, HNMT, HROB, ICAM2, ID3, IL18RAP, INKA1, IRF5, JUP, KCNC4, KIR2DL4, KMT5B, LARP1, LDLRAP1, LINC01347, LINC02363, LRRN3, LTA, MAD1L1, MBOAT2, MIAT, MRPS6, MSANTD1, N4BP2, NABP1, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SNAPC2, SNHG5, SNRPN, SPC24, SRRM2, STAB1, TACSTD2, TCAF1, TDRD9, TEX2, THEM6, TK1, TMEM116, TMEM144, TMEM183A, TMTC1, TOP2A, TSC22D1, TTC39C, UGP2, UVSSA, VNN1, ZBTB16, ZNF608, ZNF792.

For example, the following subsets from the list of 131 markers are capable of achieving an AUC > 0.85, but in most cases an AUC > 0.9, and in many cases an AUC > 0.95, for predicting development of organ dysfunction on specific days prior to development of symptoms:
1. AFDN, HROB, SRRM2, NABP1, UVSSA, KMTSB, PGA5, IRF5 (Day -1 before symptoms);
2. SLC39A8, KCNC4, AC112777.1, FOCAD, LINC02363, TMTC1, RBMS2P1, RAP1A (Day -2 before symptoms);
3. AC112777.1, FOCAD, RABGEF1, RWDD2A, FBXW9, AC141557.1, TMEM183A, HBD, CACTIN (Day -3 before symptoms);
4. NABP1, FBN2, HROB, UVSSA, SLC39A8, NUSAP1, SGSH, STAB1, SRRM2, CLN3, SNAPC2, N4BP2, CDCA7 (Day -1 before symptoms);
5. NABP1, SLC39A8, NUSAP1, CDCA7, TACSTD2, CLASP1, NEAT1, NTN3 (Day -2 before symptoms);
6. TDRD9, POLQ, C1orf226, IL18RAP, POTEG, PDXDC1, EIF1AY, RNVU1-7 (Day -3 before symptoms);
7. CXCR2P1, INKA1, BTNL8, ZNF792, TMEM116, TMEM144, PGA4, C1orf116, NIPAL3, PABPC1, VNN1, AC132942.1 (Day -1 before symptoms);
8. BTNL8, SLC39A8, ARL4A, PKIA, CCNY, ID3, HNMT, FCMR (Day -2 before symptoms);
9. TMEM116, JUP, TTC39C, FAM83A, MAD1L1, HCAR2, CD58, CCDC32 (Day -3 before symptoms);
10. SLC26A8, FBN2, NABP1, SGSH, NEAT1, ZBTB16, OIP5, PTGS2, PGD, GRAP2, BEX1, TCAF1, ATP5MD, AL133444.1 (Day -1 before symptoms);
11. SLC39A8, PAFAH2, TACSTD2, MRPS6, TK1, THEM6, SERPINA10, LRRN3 (Day -2 before symptoms);
12. TDRD9, GIMAP5, CENPE, B4GALT5, AC112777.1, ASB1, LDLRAP1, DLGAPS (Day -3 before symptoms);
13. GRAP2, SNRPN, SRRM2, PGA5, HROB, LARP1, HNMT, MBOAT2 (Day -1 before symptoms);
14. KCNC4, FHDC1, POLQ, CDCA7, CLECL1, RAP1A, CCNY, CPA3 (Day -2 before symptoms);
15. SELENOM, C7orf50, RWDD2A, ADCK2, HCAR3, AC141557.1, BAG3, TOP2A (Day -3 before symptoms);
16. NABP1, ASB1, HROB, SRRM2, OAS2, PGD, CPEB4, TSC22D1, PTGS2, ATP5MD, SNHG5, CDCA7, SNAPC2, C22orf46, CXCL16 (Day -1 before symptoms);
17. CDCA7, SLC39A8, TACSTD2, CLASP1, AC112777.1, SERPINA10, LINC01347, FCMR (Day -2 before symptoms);
18. DHRS9, PFKFB2, MSANTD1, TMEM116, UGP2, FAR2, ZNF792, LDLRAP1, ZNF608, PDXDC1, OIP5, PID1, KIR2DL4 (Day -3 before symptoms).

These subsets of markers may be optimal for a specific day before the onset of symptoms of organ dysfunction (sepsis), although may be used in a test to identify development of infection or sepsis at any point before or after onset of symptoms, whereas other subsets of markers from the list of 131 markers may also be used for providing predictions, on individual days before symptoms or multiple days before symptoms, for example other subsets of markers from the list of 131 may be capable of predicting development of infection with an AUC > 0.85, with many with an AUC >0.9 or >0.95, irrespective of the number of days prior to onset of symptoms, up to at least three days prior to onset of symptoms. For example, the subsets (signatures) of (1) AFDN, AL096803.2, ASPM, CDCA7, LARP1, NABP1, OIP5, SLC11A2, SLC39A8, SRRM2, TACSTD2, TMTC1; (2) ANKS1A, UVSSA, FBN2, GAS6, NABP1, MIAT, OPLAH, SLC11A2, SLC39A8, SPC24, TACSTD2, TDRD9, TEX2; (3) BTNL8, GRAP2, H2BC11, ID3, INKA1, SLC39A8, TMEM116, ZNF792; (4) FBN2, FCER1A, HLA-DMB, NABP1, ICAM2, LTA, MIAT, PAFAH2, SLC11A2, SLC26A8, SLC39A8, TACSTD2, TEX2; (5) AFDN, AL096803.2, BTNL8, CDCA7, FHDC1, GRAP2, ID3, INKA1, NABP1, OIP5, SLC11A2, SLC39A8, SRRM2, TACSTD2, TMEM116; (6) FBN2, FCER1A, GAS6, HLA-DMB, NABP1, ICAM2, LTA, MIAT, PAFAH2, SLC11A2, SLC39A8, SRRM2, TACSTD2, TDRD9, TEX2, UVSSA.

Key nucleic acid markers, present in multiple (2 or more) down-selected subsets for prediction of organ dysfunction versus no organ dysfunction are AC112777.1 (4), AC141557.1 (2), AFDN (3), AL096803.2 (2), ASB1 (2), ATPSMD (2), BTNL8 (4), CCNY (2), CDCA7 (7), CLASP1 (2), FBN2 (6), FCER1A (3), FCMR (2), FHDC1 (2), FOCAD (2), GAS6 (2), GRAP2 (4), HLA-DMB (3), HNMT (2), HROB (4), ICAM2 (3), ID3 (3), INKA1 (3), KCNC4 (2), LARP1 (2), LTA (3), MIAT (4), NABP1 (11), NEAT1 (2), NUSAP1 (2), OIP5 (3), PAFAH2 (4), PGA5 (2), PGD (2), POLQ (2), PTGS2 (2), RAP1A (2), RWDD2A (2), SERPINA10 (2), SGSH (2), SLC11A2 (6), SLC26A8 (3), SLC39A8 (13), SNAPC2 (2), SRRM2 (7), TACSTD2 (9), TDRD9 (4), TEX2 (4), TMEM116 (4), TMTC1 (2), UVSSA (4), ZNF792 (2).

Key markers for predicting organ dysfunction include especially SLC39A8, which occurs in 13 down-selected nucleic acid marker signatures, NABP1, which occurs in 11, TACSTD2, which occurs in 9, CDCA7, which occurs in 7, FBN2, which occurs in 6, and SLC11A2, which occurs in 6 down-selected nucleic acid marker signatures. A suitable marker set for organ dysfunction may thus include one or more of these nucleic acids, and could be a signature for example including at least 4 of these nucleic acids (such as SLC39A8, NABP1, CDCA7, FBN2; or SLC39A8, NABP1, CDCA7, TACSTD2; or SLC39A8, NABP1, FBN2, SLC11A2, which are some of the selections which appear in exemplified marker sets in the present application), or perhaps all six of these nucleic acids.

The selection of markers thus may comprise one or more of the key nucleic acid markers that recur during down-selection of subsets of markers. The at least 4 nucleic acid markers may for example comprise SLC39A8, or perhaps one or more of SLC39A8, NABP1, CDCA7, TACSTD2, FBN2, and/or SLC11A2, markers that recur in numerous subsets.

The biomarker signatures (lists of nucleic acid markers) for predicting development of organ dysfunction are all capable of achieving an AUC of at least 0.85, with the majority achieving an AUC of > 0.9 or > 0.95, which is particularly advantageous for predicting organ dysfunction, which until now has been difficult to predict, with the majority of studies in the past focussing on predicting sepsis based on the previous definition of sepsis, i.e. SIRS in response to infection, thus infection versus absence of infection alone.

The biomarker signatures of the present invention are thus especially valuable as they are capable of providing a test with high predictivity for organ dysfunction, but which may also be able to predict infection versus no infection with high predictivity, either together or separately, as part of a test for predicting sepsis, with the added value of better informing treatment and monitoring treatment of a subject.

The at least 4 nucleic acid markers to be determined in the method of the first aspect of the invention may be selected from any of the subsets, or key nucleic acid markers, that have been identified or down-selected for differentiating development of infection from no-infection, or development of organ dysfunction from non-complicated infection or no-organ dysfunction, and in any combination, from the list of 157 or 131 markers identified as highly significant to predicting sepsis.

In a second aspect, the present description, which is not part of the invention, provides a method for monitoring a subject at risk of developing infection and/or organ dysfunction and/or sepsis, the method comprising determining levels of at least four nucleic acid markers, or the products expressed by those nucleic acids, in biological samples taken from the subject at multiple time points, wherein the monitored levels of the at least four markers are used to predict development of infection and/or organ dysfunction and/or sepsis., wherein the at least four nucleic acid markers are selected from the lists consisting of:
AC005747.1, AC112777.1, AC132942.1, AC141557.1, ACVR1B, ADCK2, AFDN, AL096803.2, AL133444.1, ANKS1A, ARHGEF40, ARL4A, ASB1, ASPM, ATP5MD, B4GALT5, BAG3, BEX1, BTNL8, C1orf116, C1orf226, C22orf46, C7orf50, CACTIN, CBS, CCDC32, CCNY, CD177P1, CD58, CDCA7, CDH23, CENPE, CGTLC4P, CLASP1, CLECL1, CLN3, CPA3, CPEB4, CXCL16, CXCR2P1, DHRS9, DLGAP5, EIF1AY, EIF4G3, FAM83A, FAR2, FBN2, FBXL18, FBXW9, FCER1A, FCMR, FHDC1, FOCAD, FOXI3, GAS6, GAS7, GGTLC4P, GIMAP5, GRAP2, H2BC11, HBD, HCAR2, HCAR3, HIPK2, HLA-DMB, HNMT, HROB, ICAM2, ID3, IL18RAP, INKA1, IRF5, IRX3, JUP, KCNC4, KIR2DL4, KMT5B, LARP1, LDLR, LDLRAP1, LINC01347, LINC02363, LMNA, LRRN3, LTA, MAD1L1, MBOAT2, MIAT, MRPS6, MSANTD1, MTRR, N4BP2, NABP1, NCAPG2, NCOR2, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SLC51A, SMG1, SNAPC2, SNHG5, SNRPN, SPC24, SPTLC2, SQLE, SRRM2, STAB1, TACSTD2, TCAF1, TDRD9, TEX2, THBS3, THEM6, TK1, TLE1, TMEM116, TMEM144, TMEM183A, TMTC1, TOP2A, TSC22D1, TTC39C, UGP2, UVSSA, VNN1, VPS9D1, ZBTB16, ZNF608, ZNF792, or
AC112777.1, AC132942.1, AC141557.1, ADCK2, AFDN, AL096803.2, AL133444.1, ANKS1A, ARL4A, ASB1, ASPM, ATP5MD, B4GALT5, BAG3, BEX1, BTNL8, C1orf116, C1orf226, C22orf46, C7orf50, CACTIN, CCDC32, CCNY, CD58, CDCA7, CENPE, CLASP1, CLECL1, CLN3, CPA3, CPEB4, CXCL16, CXCR2P1, DHRS9, DLGAP5, EIF1AY, FAM83A, FAR2, FBN2, FBXW9, FCER1A, FCMR, FHDC1, FOCAD, GAS6, GIMAP5, GRAP2, H2BC11, HBD, HCAR2, HCAR3, HLA-DMB, HNMT, HROB, ICAM2, ID3, IL18RAP, INKA1, IRF5, JUP, KCNC4, KIR2DL4, KMTSB, LARP1, LDLRAP1, LINC01347, LINC02363, LRRN3, LTA, MAD1L1, MBOAT2, MIAT, MRPS6, MSANTD1, N4BP2, NABP1, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SNAPC2, SNHG5, SNRPN, SPC24, SRRM2, STAB1, TACSTD2, TCAF1, TDRD9, TEX2, THEM6, TK1, TMEM116, TMEM144, TMEM183A, TMTC1, TOP2A, TSC22D1, TTC39C, UGP2, UVSSA, VNN1, ZBTB16, ZNF608, ZNF792.

The biomarkers disclosed therein, or subsets thereof, are particularly advantageous for use in a test for monitoring a subject at risk over several days, which could incorporate different marker sets to identify the most likely day before symptoms, such as Day-1, Day-2, or Day-3 before symptoms, and whether infection and/or organ dysfunction is likely, and thereby consequently inform the best course of treatment to prevent or treat infection/organ dysfunction. The monitoring may comprise comparing dynamic changes in quantitation or rates of change of the biomarkers to derive predictors, such as for whether and when a subject may develop sepsis. For example, monitoring may comprise interrogation of biomarker velocity, such as its rate of change over time. An AUC of 0.9 and above for predicting organ dysfunction is achievable with nucleic acid markers subsets form the list of 157 or 131 markers, which is particularly good for predicting organ dysfunction versus absence of organ dysfunction, as no biomarker set to date has been identified that is particularly directed to organ dysfunction (and especially not with such a high predictivity), since most studies on sepsis have used the previous definition of sepsis, which was concerned with infection alone, and not organ dysfunction, and especially not an approach that could evaluate both infection and organ dysfunction with a single set of biomarkers, or different groups of biomarkers for each of infection and organ dysfunction. Particular subsets of markers are as for the first aspect of the present invention.

In a third aspect, the present description, which is not part of the invention, provides a kit for predicting development of infection and/or organ dysfunction and/or sepsis in a subject, said kit comprising reagents and/or systems for determining levels of at least four markers in a biological sample from the subject, wherein the at least four markers are selected from the lists consisting of:
AC005747.1, AC112777.1, AC132942.1, AC141557.1, ACVR1B, ADCK2, AFDN, AL096803.2, AL133444.1, ANKS1A, ARHGEF40, ARL4A, ASB1, ASPM, ATP5MD, B4GALT5, BAG3, BEX1, BTNL8, C1orf116, C1orf226, C22orf46, C7orf50, CACTIN, CBS, CCDC32, CCNY, CD177P1, CD58, CDCA7, CDH23, CENPE, CGTLC4P, CLASP1, CLECL1, CLN3, CPA3, CPEB4, CXCL16, CXCR2P1, DHRS9, DLGAP5, EIF1AY, EIF4G3, FAM83A, FAR2, FBN2, FBXL18, FBXW9, FCER1A, FCMR, FHDC1, FOCAD, FOXI3, GAS6, GAS7, GGTLC4P, GIMAP5, GRAP2, H2BC11, HBD, HCAR2, HCAR3, HIPK2, HLA-DMB, HNMT, HROB, ICAM2, ID3, IL18RAP, INKA1, IRF5, IRX3, JUP, KCNC4, KIR2DL4, KMT5B, LARP1, LDLR, LDLRAP1, LINC01347, LINC02363, LMNA, LRRN3, LTA, MAD1L1, MBOAT2, MIAT, MRPS6, MSANTD1, MTRR, N4BP2, NABP1, NCAPG2, NCOR2, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SLC51A, SMG1, SNAPC2, SNHG5, SNRPN, SPC24, SPTLC2, SQLE, SRRM2, STAB1, TACSTD2, TCAF1, TDRD9, TEX2, THBS3, THEM6, TK1, TLE1, TMEM116, TMEM144, TMEM183A, TMTC1, TOP2A, TSC22D1, TTC39C, UGP2, UVSSA, VNN1, VPS9D1, ZBTB16, ZNF608, ZNF792, or
AC112777.1, AC132942.1, AC141557.1, ADCK2, AFDN, AL096803.2, AL133444.1, ANKS1A, ARL4A, ASB1, ASPM, ATP5MD, B4GALT5, BAG3, BEX1, BTNL8, C1orf116, C1orf226, C22orf46, C7orf50, CACTIN, CCDC32, CCNY, CD58, CDCA7, CENPE, CLASP1, CLECL1, CLN3, CPA3, CPEB4, CXCL16, CXCR2P1, DHRS9, DLGAP5, EIF1AY, FAM83A, FAR2, FBN2, FBXW9, FCER1A, FCMR, FHDC1, FOCAD, GAS6, GIMAP5, GRAP2, H2BC11, HBD, HCAR2, HCAR3, HLA-DMB, HNMT, HROB, ICAM2, ID3, IL18RAP, INKA1, IRF5, JUP, KCNC4, KIR2DL4, KMTSB, LARP1, LDLRAP1, LINC01347, LINC02363, LRRN3, LTA, MAD1L1, MBOAT2, MIAT, MRPS6, MSANTD1, N4BP2, NABP1, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SNAPC2, SNHG5, SNRPN, SPC24, SRRM2, STAB1, TACSTD2, TCAF1, TDRD9, TEX2, THEM6, TK1, TMEM116, TMEM144, TMEM183A, TMTC1, TOP2A, TSC22D1, TTC39C, UGP2, UVSSA, VNN1, ZBTB16, ZNF608, ZNF792.

The subject is most likely a human, but may also be an animal, and the biological sample is most likely a blood or serum sample.

Particular subsets of markers are as for the first aspect of the present invention.

The kit disclosed therein may comprise means for detecting levels of a nucleic acid or nucleic acid product. Although nucleic acid expression may be determined by detecting the presence of nucleic acid products including proteins and peptides, such processes may be complex. In a particular embodiment, the means comprises means for detecting a nucleic acid, for example RNA, such as mRNA, or means for detecting a product expressed by the nucleic acid, such as a protein.

The reagents or systems may include use of recognition elements, or microarray based methods. Thus in a particular embodiment, the kit of the invention comprises a microarray on which are immobilised probes suitable for binding to RNA expressed by each nucleic acid of a biomarker signature. Means for detecting a protein may comprise an antibody, which may be a fluorescently-labelled antibody, and may comprise protein recognition elements on a microarray, or other suitable platform, such as lateral flow strips.

In an alternative embodiment, the kit comprises at least some of the reagents suitable for carrying out amplification of nucleic acids of the biomarker signature, or regions thereof.

In one embodiment the reagents or systems use real-time (RT) polymerase chain reaction (PCR). In such cases, the reagents may comprise primers for amplification of said nucleic acids or regions thereof. The kits may further comprise labels in particular fluorescent labels and/or oligonucleotide probes to allow the PCR to be monitored in real-time using any of the known assays, such as TaqMan, LUX, etc. The kits may also contain reagents such as buffers, enzymes, salts such as MgCl etc. required for carrying out a nucleic acid amplification reaction. The reagents, especially for nucleic acid amplification, may comprise for example one or more of fluorescently-labelled oligonucleotide probes or fluorescently-labelled primers, wherein the fluorescently-labelled oligonucleotide probes or fluorescently-labelled primers may consist of probes and primers each capable of specific binding and detection of nucleic acid products of the least 4 markers.

The methods of the first or second aspect may advantageously be computer-implemented to handle the complexity in monitoring and analysis of the numerous biomarkers, and their respective relationships to each other. Such a computer-implemented invention could enable a yes/no answer as to whether infection and/or organ dysfunction and/or sepsis is likely to develop, or at least provide an indication of how likely the development is.

The method preferably uses mathematical tools and/or algorithms to monitor and assess expression of the biomarkers (the nucleic acid markers or products thereof) both qualitatively and quantitatively. The tools could in particular include support vector machine (SVM) algorithms, decision trees, random forests, artificial neural networks, quadratic discriminant analysis, and Bayes classifiers. In one embodiment the data from monitoring all biomarkers in the biomarker signature is assessed by means of an artificial neural network.

In one embodiment of the first or second aspect the method is a computer-implemented method wherein the monitoring, measuring and/or detecting comprises producing quantitative, and optionally qualitative, data for all markers, inputting said data into an analytical process on the computer, using at least one mathematical method, that may compare the data with reference data, and producing an output from the analytical process which provides a prediction for the likelihood of developing infection and/or organ dysfunction and/or sepsis, or enables monitoring of the condition. The reference data may include data from healthy subjects, subjects diagnosed with sepsis (organ dysfunction), subjects with infection, and subjects with SIRS, but no infection.

The output from the analytical process may enable the time to onset of symptoms to be predicted, such as 1, 2, or 3 days prior to onset of symptoms, and consequently may be particularly valuable and useful to a medical practitioner in suggesting a course of treatment, especially when the choice of course of treatment is dependent on the progression of the disease. The method may also enable monitoring of the success of any treatment, assessing whether the likelihood of onset of symptoms decreases over the course of treatment.

In a fourth aspect, the present description, which is not part of the invention, provides an apparatus for analysis of a biological sample from a subject to predict or monitor the development of sepsis comprising means for monitoring, measuring or detecting the expression of at least four markers in a biological sample from the subject, wherein the at least four markers are selected from the lists consisting of:
AC005747.1, AC112777.1, AC132942.1, AC141557.1, ACVR1B, ADCK2, AFDN, AL096803.2, AL133444.1, ANKS1A, ARHGEF40, ARL4A, ASB1, ASPM, ATP5MD, B4GALT5, BAG3, BEX1, BTNL8, C1orf116, C1orf226, C22orf46, C7orf50, CACTIN, CBS, CCDC32, CCNY, CD177P1, CD58, CDCA7, CDH23, CENPE, CGTLC4P, CLASP1, CLECL1, CLN3, CPA3, CPEB4, CXCL16, CXCR2P1, DHRS9, DLGAP5, EIF1AY, EIF4G3, FAM83A, FAR2, FBN2, FBXL18, FBXW9, FCER1A, FCMR, FHDC1, FOCAD, FOXI3, GAS6, GAS7, GGTLC4P, GIMAP5, GRAP2, H2BC11, HBD, HCAR2, HCAR3, HIPK2, HLA-DMB, HNMT, HROB, ICAM2, ID3, IL18RAP, INKA1, IRF5, IRX3, JUP, KCNC4, KIR2DL4, KMT5B, LARP1, LDLR, LDLRAP1, LINC01347, LINC02363, LMNA, LRRN3, LTA, MAD1L1, MBOAT2, MIAT, MRPS6, MSANTD1, MTRR, N4BP2, NABP1, NCAPG2, NCOR2, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SLC51A, SMG1, SNAPC2, SNHG5, SNRPN, SPC24, SPTLC2, SQLE, SRRM2, STAB1, TACSTD2, TCAF1, TDRD9, TEX2, THBS3, THEM6, TK1, TLE1, TMEM116, TMEM144, TMEM183A, TMTC1, TOP2A, TSC22D1, TTC39C, UGP2, UVSSA, VNN1, VPS9D1, ZBTB16, ZNF608, ZNF792, or
AC112777.1, AC132942.1, AC141557.1, ADCK2, AFDN, AL096803.2, AL133444.1, ANKS1A, ARL4A, ASB1, ASPM, ATP5MD, B4GALT5, BAG3, BEX1, BTNL8, C1orf116, C1orf226, C22orf46, C7orf50, CACTIN, CCDC32, CCNY, CD58, CDCA7, CENPE, CLASP1, CLECL1, CLN3, CPA3, CPEB4, CXCL16, CXCR2P1, DHRS9, DLGAP5, EIF1AY, FAM83A, FAR2, FBN2, FBXW9, FCER1A, FCMR, FHDC1, FOCAD, GAS6, GIMAP5, GRAP2, H2BC11, HBD, HCAR2, HCAR3, HLA-DMB, HNMT, HROB, ICAM2, ID3, IL18RAP, INKA1, IRF5, JUP, KCNC4, KIR2DL4, KMT5B, LARP1, LDLRAP1, LINC01347, LINC02363, LRRN3, LTA, MAD1L1, MBOAT2, MIAT, MRPS6, MSANTD1, N4BP2, NABP1, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SNAPC2, SNHG5, SNRPN, SPC24, SRRM2, STAB1, TACSTD2, TCAF1, TDRD9, TEX2, THEM6, TK1, TMEM116, TMEM144, TMEM183A, TMTC1, TOP2A, TSC22D1, TTC39C, UGP2, UVSSA, VNN1, ZBTB16, ZNF608, ZNF792,
and means for analysis of data produced from the means for monitoring, measuring or detecting, such as a computer comprising an appropriate mathematical model to analyse the data, such as an artificial neural network, and means for providing an output from the analysis which output provides a prediction of the likelihood of an animal having sepsis, or an output to enable monitoring of infection and/or organ dysfunction and/or sepsis, which output could also be provided by an appropriately programmed computer.

All subsets of markers (biomarker signatures) of the different aspects of the present invention may be used to monitor and/or predict the response of a subject to a particular therapeutic agent, such as a sepsis targeting drug or an antibiotic. For example the expression of particular nucleic acid markers, which may for example be elevated in a subject on a course to develop sepsis, or having already developed sepsis, could be monitored to establish whether the levels are returning to the levels expected for a subject without, or unlikely to develop, sepsis, which could be an indication of the therapeutic agent successfully treating the subject. A therapeutic agent may be one targeted to particular subsets of markers in an attempt to treat the subject, and indeed the choice of therapeutic agent to be used in a subject may be determined by the expression of specific nucleic acid markers which are most affected, or differ most from a control or from a patient not predicted to develop sepsis, as a result of developing sepsis. For example, the elevation of certain markers may suggest the use of one therapeutic agent, whereas elevation of a different subset of markers, may suggest use of another therapeutic agent.

Any feature in one aspect of the description may be applied to any other aspects of the invention, in any appropriate combination. In particular, method aspects may be applied to use, kit and system aspects and vice versa. The description extends to methods, uses, kits or systems substantially as herein described, with reference to the Example(s).

In all aspects, the invention may comprise, consist essentially of, or consist of any feature or combination of features.

### Example - Development of a predictive panel of pre-symptomatic biomarkers for infection, organ dysfunction, and thereby sepsis

The aim of this program of work was to develop a predictive panel of pre-symptomatic biomarkers for infection and organ dysfunction (sepsis), through comprehensive analysis of the host transcriptome, sourced from blood samples from human patients collected prior to the clinical onset of infection or organ dysfunction, and to develop biomarker signatures that may indicate whether and when clinical symptoms will arise. In so doing it would yield a suitably powered bioinformatic model for differentiating sepsis patients from control patients based on transcriptomic biomarker signatures. In turn, this will assist in the development of RT-PCR methods for infection and organ dysfunction prediction, where this capability should provide timely diagnosis and treatment when medical countermeasures are most effective.

We used microarray technology to obtain gene (nucleic acid) expression data of samples derived from pre-symptomatic patients and control patient samples. An unsupervised bioinformatic approach was used to identify prognostic transcriptomic expression patterns that characterize infection and organ dysfunction before the onset of clinical symptoms.

The Applicant conducted a large prospective, multicenter study in patients undergoing elective major surgery, with daily blood sampling and data recording commencing before the operation and continuing for up to a week after, to enable pre-symptomatic identification of patients developing infection complicated or not by new-onset organ dysfunction (sepsis). Crucially, there was a clinical adjudication panel who independently examined clinical and laboratory data to identify patients with definite infection (± sepsis). Samples from these patients enabled accurate comparison of microarray data against cohorts of age-, sex- and procedure-matched patients with non-infective systemic inflammation (SIRS) or an uncomplicated postoperative course. Nucleic acid expression signatures measured in blood samples could identify patients developing infection up to three days prior to clinical presentation, and could differentiate between patients developing uncomplicated infection or sepsis (organ dysfunction).

### Patient recruitment

Elective surgery patients were prospectively recruited into this study between November 2007 and February 2017. Patients were enrolled if they gave informed consent, were between 18-80 years of age and undergoing an elective high-risk surgical procedure that placed them at an increased risk of infection ± sepsis. Recruitment occurred at seven centres in the UK and one in Germany.

Clinical and blood samples were collected from 4,385 patients undergoing high-risk elective surgery. The total number of sample vials received was 72,734, and subsequent sub-aliquoting of key samples for further analysis generated a further 81,800 vials. 155 patients were adjudicated by the clinical advisory panel (CAP) to have definite post-operative infection. Samples from 63 of these patients, of whom 37 developed new organ dysfunction (sepsis), underwent detailed analyses.

Differentially expressed genes (DEGs) were identified through comparisons of samples that were (1) from patients who became infected versus patients who did not become infected, (2) patients who developed organ dysfunction (sepsis) versus patients who developed non-complicated infection (i.e. no organ dysfunction), and (3) patients who developed organ dysfunction (sepsis) versus all other patients in the study (i.e. those that either did not become infected, had non-complicated infection, or patients displaying SIRS criteria).

In microarray analysis, samples from (1) patients who became infected were compared against age-, sex- and procedure-matched patients with an uncomplicated, non-infected, non-inflamed course, (2) patients who developed organ dysfunction were compared against age-, sex- and procedure-matched patients who developed a non-complicated infection, and (3) patients who developed organ dysfunction (sepsis) were compared against age-, sex- and procedure-matched other patients in the study (i.e. those who did not become infected, or had non-complicated infection, or displayed non-specific SIRS criteria). Microarray analysis was performed on blood samples taken up to 3 days (Days -1, -2 and -3) prior to diagnosis, of either infection or organ dysfunction as applicable, and was undertaken using different combinations of the DEGs identified, as detailed in the previous paragraph, to ensure the analysis was tuned to answer specific questions. The Applicant was especially concerned with identifying biomarkers which could differentiate those patients likely to develop organ dysfunction, over and above all other patients, thus the reason behind identifying different subsets of DEGs.

### Sampling

Blood sample collection occurred once between 1-7 days before surgery and then daily postoperatively until seven days, hospital discharge (if earlier), or diagnosis of infection or sepsis by the treating clinician.

### Sepsis patient selection process

Initial diagnosis of infection or sepsis was based on the treating clinician's interpretation of clinical and laboratory markers using the then-extant 'Sepsis-2' definition of sepsis. This described *'sepsis'* as suspected or confirmed infection with two or more systemic inflammatory response syndrome (SIRS) criteria, and *'severe sepsis'* as sepsis plus new-onset organ dysfunction. The new sepsis definition (Sepsis-3) published in February 2016 rebadged *'sepsis'* as infection plus new organ dysfunction identified by a rise of ≥2 points from one day to the next of the patient's SOFA score. To keep in line with modern nomenclature, subsequent analyses and descriptors apply the new definition.

As initial diagnosis of infection and sepsis is often based on clinical judgement and before any microbiological confirmation, the CAP was formed to adjudicate cases labelled as postoperative infection. A minimum of five specialists in intensive care or microbiology independently reviewed clinical, laboratory and imaging results to give a high confidence diagnosis of infection. These patients were allocated to either an infection group, a non-infective systemic inflammation (SIRS) group, or an uncomplicated post-operative recovery group assuming no significant non-infective issues arose (e.g. hemorrhage, myocardial infarction). The infection group was subsequently divided into 2 sub-groups, those with or without organ dysfunction as defined by an increase of the patient's SOFA score by 2 or more from one day to the next.

### Microarray analysis

For each sample analysed, globin-reduced RNA (GlobinClear^{™}, ThermoFisher) was prepared from total RNA. RNA integrity was measured using an Agilent Bioanalyzer 2100 (Town, State) and concentration using a NanoQuant^{™} (Tecan, Town). cRNA was prepared by amplification and labeling using the Illumina^{®} TotalPrep^{™} RNA Amplification Kit (ThermoFisher) and hybridized to Human HT-12v4 Beadarrays (Illumina^{®},Place, State). An Illumina^{®} HighScanHQ^{™} then imaged each chip with resulting intensities indicating the expression level of each probe's corresponding gene.

The Illumina^{®} Human HT-12v4 beadarrays were preprocessed and background corrected using GenomeStudio^{™} Software v2011.1 (Illumina^{®}). To obtain genes with the greatest evidence of differential expression, a linear model fit was applied for each gene using the limma package (Doi: 10.1093/nar/gkv007: Ritchie, M.E. et al, Nucleic Acids Res., 2015, 43(7)). Datasets include patient groups with or without infection up to three days before diagnosis of infection. Data obtained from non-infected patients were used as a reference. A false positive rate of 0.05 with FDR correction and a fold change greater than 1.3 was taken as the level of significance.

### Feature selection and learning of predictive models

To improve the performance metric of the predictive models a two-step feature selection was performed. First, the Boruta algorithm, a wrapper method based on Random forest was used for selection of relevant features in the data set. Then a new randomized feature (shadow feature) was added for each feature in the dataset. The classifier was then trained with the dataset and the importance of each feature calculated. Real features that have a significantly higher z-score than the best shadow feature are called relevant features. The Boruta algorithm was applied in a 5-fold cross-validation, repeated 25 times. A feature identified as relevant in at least one model was considered for further evaluation. As a second step, backward elimination was used to determine those features with the most discriminatory power for a particular classification problem. Starting with all relevant features in a 5 fold cross validation repeated 25 times the importance of features was calculated. This loop was reiterated until a maximum of the assessment index area under the curve (AUC) was found. In each iteration step, the feature with the least importance was removed.

### Results

### Microarray Gene Expression Analysis of Infected Patients

Transcriptomic sequencing was carried out on samples from 58 patients taken over the three days preceding clinical presentation of post-operative infection and compared to 55 matched healthy postoperative controls. Overall 2337 differentially expressed genes (DEGs) with fold change of at least 1.2 between infection and control were identified. Of 1500 DEGs with the highest fold change, 58% (870 DEGs) were up-regulated and 42% (630) downregulated.

DEGs between infection and no infection (control) patients/samples with a fold change of at least 1.3 were identified, and numbered 1111 nucleic acids. DEGs between organ dysfunction and non-complicated infection patients/samples with a fold change of at least 1.3 were identified, and numbered 447 nucleic acids. DEGs between organ dysfunction patients/samples, and all other patients/samples (which did not go on to develop organ dysfunction) were identified, and numbered 924 nucleic acids.

### Classification of development of infection and organ dysfunction based on microarray expression

A random forest-based algorithm was used to classify differential gene expression on Days -1, -2, or - 3 prior to infection diagnosis or organ dysfunction (sepsis) diagnosis against respective controls. Random forest reports the most important genes to reach performance next to statistical metrics.

### Infection versus no infection (control) using the 1111 nucleic acids identified to be differentially expressed between infection and no infection patients/samples (with a fold change of at least 1.3).

The best identified classification for Day -1 (based on 54 infection plus and 51 infection minus samples) reached an Area under the ROC Curve (AUC) of 0.985 and a positive predictive value (PPV) of 0.927 for a set of eight genes (SPTLC2, LDLR, EIF4G3, FBXL18, GGTLC4P, ARHGEF40, CD177P1, CDH23). The best identified classification for Day -2 (based on 45 infection plus and 42 infection minus samples) achieved an AUC of 0.982 (PPV 0.944) using a different set of eight genes from the overall set (*SLC51A, B4GALT5, THBS3, MTRR, IRX3, CBS, TOP2A, NCAPG2*), and the best classification for Day -3 (based on 35 infection plus and 33 infection minus samples) achieved an AUC of 0.989 (PPV 0.952) using again eight different genes (CGTLC4P, *GAS7, NCOR2,* FOXI3, *TLE1, SQLE, SMG1, LMNA).*

Classification of infection was repeated with a requirement for the same set of genes used by the random forest classifier for each day (Days -1, -2 and -3) prior to infection diagnosis. The best performing classifiers by random forest required 12 gene features and achieved AUC values of 0.959 for Day -1 (PPV 0.866), 0.924 for Day -2 (PPV 0.831), and 0.889 for Day -3 (PPV 0.815). The genes were ACVR1B, ANKS1A, B4GALT5, VPS9D1, EIF4G3, FBXL18, AC005747.1, HIPK2, LDLR, MIAT, OPLAH, SPTLC2.

### Organ dysfunction (sepsis) versus non-complicated infection (control) using the 1111 nucleic acids identified to be differentially expressed between infection and no infection patients/samples (with a fold change of at least 1.3).

The best identified classification for Day -1 (based on 31 organ dysfunction plus and 23 non-complicated infection samples) achieved an AUC of 0.956 (PPV 0.900) based on eight genes (*AFDN, HROB, SRRM2, NABP1, UVSSA, KMTSB, PGA5, IRF5).* The best classification for Day -2 (based on 26 organ dysfunction plus and 19 non-complicated infection samples) was achieved with eight genes *(SLC39A8, KCNC4, AC112777.1, FOCAD, LINC02363, TMTC1, RBMS2P1, RAP1A*), yielding an AUC of 0.975 (PPV 0.958). The classification performance for Day -3 (based on 20 organ dysfunction plus and 15 non-complicated infection samples) yielded an AUC of 0.990 (PPV 0.970) based on nine genes *(AC112777.1, FOCAD,* RABGEF1, RWDD2A, FBXW9, AC141557.1, TMEM183A, HBD, CACTIN).

Following the same procedure as for development of infection, another classification model set based on random forest was created that required the same gene set for each separate day. AUC values of 0.861 (PPV 0.798) for Day -1, 0.910 (PPV 0.838) for Day -2 and 0.739 (PPV 0.712) for Day -3 were achieved with twelve genes (*AFDN, AL096803.2, ASPM, CDCA7, LARP1, NABP1, OIP5, SLC11A2, SLC39A8, SRRM2, TACSTD2, TMTC1).*

At least some of these predictive AUC values were the highest that the Applicant had been able to observe for predicting organ dysfunction, and these were selected on DEGs derived from infection versus no-infection. It was hypothesized that these AUCs may indeed be able to be improved if the starting point was more relevant to the question, and thus DEGs between those developing organ dysfunction versus non-complicated infection, or even developing organ dysfunction versus all other patients/samples (i.e. all those not developing organ dysfunction, i.e. sepsis).

It was first decided however to identify the best classifying biomarker sets for developing organ dysfunction versus all other patients/samples using the 1111 DEGS, so this could be used for later comparisons.

### Organ dysfunction (sepsis) versus all others (control) using the 1111 nucleic acids identified to be differentially expressed between infection and no infection patients/samples (with a fold change of at least 1.3).

The best identified for classification for Day -1 (based on 31 organ dysfunction plus and 74 other samples) achieved an AUC of 0.963 (PPV 0.942) based on thirteen genes (NABP1, FBN2, HROB, UVSSA, SLC39A8, NUSAP1, SGSH, STAB1, SRRM2, CLN3, SNAPC2, N4BP2, CDCA7). The best classification for Day -2 (based on 26 organ dysfunction plus and 61 other samples) was achieved with eight genes (NABP1, SLC39A8, NUSAP1, CDCA7, TACSTD2, CLASP1, NEAT1, NTN3), yielding an AUC of 0.968 (PPV 0.966). The classification performance for Day -3 (based on 20 organ dysfunction plus and 48 other samples) yielded an AUC of 0.980 (PPV 0.954) based on eight genes (TDRD9, POLQ, C1orf226, IL18RAP, POTEG, PDXDC1, EIF1AY, RNVU1-7).

Following the same procedure as for development of infection, another classification model set based on random forest was created that required the same gene set for each separate day. AUC values of 0.904 (PPV 0.802) for Day -1, 0.918 (PPV 0.839) for Day -2 and 0.874 (PPV 0.839) for Day -3 were achieved with thirteen genes (ANKS1A, UVSSA, FBN2, GAS6, NABP1, MIAT, OPLAH, SLC11A2, SLC39A8, SPC24, TACSTD2, TDRD9, TEX2).

### Organ dysfunction (sepsis) versus non-complicated infection (control) using the 447 nucleic acids identified to be differentially expressed between patients developing organ dysfunction and non-complicated infection patients/samples (with a fold change of at least 1.3).

The best identified for classification for Day -1 (based on 31 organ dysfunction plus and 23 non-complicated infection samples) achieved an AUC of 0.910 (PPV 0.857) based on twelve genes (CXCR2P1, INKA1, BTNL8, ZNF792, TMEM116, TMEM144, PGA4, C1orf116, NIPAL3, PABPC1, VNN1, AC132942.1). The best classification for Day -2 (based on 26 organ dysfunction plus and 19 non-complicated infection samples) was achieved with eight genes (BTNL8, SLC39A8, ARL4A, PKIA, CCNY, ID3, HNMT, FCMR), yielding an AUC of 0.904 (PPV 0.836). The classification performance for Day -3 (based on 20 organ dysfunction plus and 15 non-complicated infection samples) yielded an AUC of 0.999 (PPV 0.994) based on eight genes (TMEM116, JUP, TTC39C, FAM83A, MAD1L1, HCAR2, CD58, CCDC32).

Following the same procedure as for development of infection, another classification model set based on random forest was created that required the same gene set for each separate day. AUC values of 0.837 (PPV 0.776) for Day -1, 0.799 (PPV 0.752) for Day -2 and 0.813 (PPV 0.736) for Day -3 were achieved with eight genes (BTNL8, GRAP2, H2BC11, ID3, INKA1, SLC39A8, TMEM116, ZNF792).

Surprisingly, using the 447 DEGs which would appear to be the most appropriate starting point for identifying the best classifying marker sets of nucleic acids for providing a predicting or organ dysfunction versus non-complicated infection, the identified marker sets generally had lower AUCs than from use of the 1111 DEGs for differentiated infection from no infection.

### Organ dysfunction (sepsis) versus all others (control) using the 924 nucleic acids identified to be differentially expressed between patients developing organ dysfunction versus all other patients/samples (with a fold change of at least 1.3).

The best identified for classification for Day -1 (based on 31 organ dysfunction plus and 74 other samples) achieved an AUC of 0.961 (PPV 0.896) based on fourteen genes (SLC26A8, FBN2, NABP1, SGSH, NEAT1, ZBTB16, OIP5, PTGS2, PGD, GRAP2, BEX1, TCAF1, ATP5MD, AL133444.1). The best classification for Day -2 (based on 26 organ dysfunction plus and 61 other samples) was achieved with eight genes (SLC39A8, PAFAH2, TACSTD2, MRPS6, TK1, THEM6, SERPINA10, LRRN3), yielding an AUC of 0.960 (PPV 0.929). The classification performance for Day -3 (based on 20 organ dysfunction plus and 48 other samples) yielded an AUC of 0.986 (PPV 0.987) based on eight genes (TDRD9, GIMAPS, CENPE, B4GALT5, AC112777.1, ASB1, LDLRAP1, DLGAP5).

Following the same procedure as for development of infection, another classification model set based on random forest was created that required the same gene set for each separate day. AUC values of 0.896(PPV 0.794) for Day -1, 0.926 (PPV 0.900) for Day -2 and 0.885 (PPV 0.748) for Day -3 were achieved with thirteen genes (FBN2, FCER1A, HLA-DMB, NABP1, ICAM2, LTA, MIAT, PAFAH2, SLC11A2, SLC26A8, SLC39A8, TACSTD2, TEX2).

Although there was some improvement in AUCs over use of the 1111 genes for infection versus no infection, the improvement was marginal. It was thus decided to use a combination of the DEGs from each of the analyses to consider whether this could improve the predictivity of the best classifying marker sets. In total from each of the analyses for DEGs, taking into account overlapping genes, there were 1453 genes in a sum of DEGs between infection versus no infection, and DEGs between organ dysfunction versus non-complicated infection, and 1649 genes in a sum of DEGs between infection versus no infection, DEGs between organ dysfunction versus non-complicated infection, and DEGs between organ dysfunction and all other patients/samples.

### Organ dysfunction (sepsis) versus non-complicated infection (control) using the total 1453 nucleic acids differentially expressed (with a fold change of at least 1.3).

The best identified for classification for Day -1 (based on 31 organ dysfunction plus and 23 non-complicated infection samples) achieved an AUC of 0.952 (PPV 0.915) based on eight genes (GRAP2, SNRPN, SRRM2, PGA5, HROB, LARP1, HNMT, MBOAT2). The best classification for Day -2 (based on 26 organ dysfunction plus and 19 non-complicated infection samples) was achieved with eight genes (KCNC4, FHDC1, POLQ, CDCA7, CLECL1, RAP1A, CCNY, CPA3), yielding an AUC of 0.988 (PPV 0.955). The classification performance for Day -3 (based on 20 organ dysfunction plus and 15 non-complicated infection samples) yielded an AUC of 0.999 (PPV 0.995) based on eight genes (SELENOM, C7orf50, RWDD2A, ADCK2, HCAR3, AC141557.1, BAG3, TOP2A).

Following the same procedure as for development of infection, another classification model set based on random forest was created that required the same gene set for each separate day. AUC values of 0.880 (PPV 0.830) for Day -1, 0.901 (PPV 0.867) for Day -2 and 0.819 (PPV 0.760) for Day -3 were achieved with fifteen genes (AFDN, AL096803.2, BTNL8, CDCA7, FHDC1, GRAP2, ID3, INKA1, NABP1, OIP5, SLC11A2, SLC39A8, SRRM2, TACSTD2, TMEM116).

The biomarker sets identified through this approach provided overall the best predictivities and highest AUCs obtained for prediction of organ dysfunction versus non-complicated infection, for separate days and all days. The final analysis was thus to use the total of 1649 DEGs from all analyses, for differentiating organ dysfunction from all other patients/samples.

### Organ dysfunction (sepsis) versus all others (control) using the total 1649 nucleic acids differentially expressed (with a fold change of at least 1.3).

The best identified for classification for Day -1 (based on 31 organ dysfunction plus and 74 other samples) achieved an AUC of 0.973 (PPV 0.954) based on fifteen genes (NABP1, ASB1, HROB, SRRM2, OAS2, PGD, CPEB4, TSC22D1, PTGS2, ATPSMD, SNHG5, CDCA7, SNAPC2, C22orf46, CXCL16). The best classification for Day -2 (based on 26 organ dysfunction plus and 61 other samples) was achieved with eight genes (CDCA7, SLC39A8, TACSTD2, CLASP1, AC112777.1, SERPINA10, LINC01347, FCMR), yielding an AUC of 0.971 (PPV 0.934). The classification performance for Day -3 (based on 20 organ dysfunction plus and 48 other samples) yielded an AUC of 0.992 (PPV 0.994) based on thirteen genes (DHRS9, PFKFB2, MSANTD1, TMEM116, UGP2, FAR2, ZNF792, LDLRAP1, ZNF608, PDXDC1, OIP5, PID1, KIR2DL4).

Following the same procedure as for development of infection, another classification model set based on random forest was created that required the same gene set for each separate day. AUC values of 0.905 (PPV 0.823) for Day -1, 0.928 (PPV 0.857) for Day -2 and 0.887 (PPV 0.814) for Day -3 were achieved with sixteen genes (FBN2, FCER1A, GAS6, HLA-DMB, NABP1, ICAM2, LTA, MIAT, PAFAH2, SLC11A2, SLC39A8, SRRM2, TACSTD2, TDRD9, TEX2, UVSSA).

The biomarker sets identified through this approach provided overall the best predictivities and highest AUCs obtained for prediction of organ dysfunction versus all other patients/samples, for separate days and all days.

### Performance parameters of organ dysfunction (sepsis) versus all others (control) for groups of nucleic acids from the 16 biomarker signature identified for all days from using the total 1649 nucleic acids differentially expressed (with a fold change of at least 1.3), as a function of the number of genes used.

**Table 1. Performance parameters for organ dysfunction versus all others using the 16 biomarker signature of FBN2, FCER1A, GAS6, HLA-DMB, NABP1, ICAM2, LTA, MIAT, PAFAH2, SLC11A2, SLC39A8, SRRM2, TACSTD2, TDRD9, TEX2, UVSSA, and subsets thereof down to 2 biomarkers. The following parameters are given: AUC-Area Under the Curve, ACC- Accuracy, PPV-Positive Predictive Value, NPV-Negative Predictive Value.**

| | **Day -1 (31 / 74)** | | | | **Day -2 (26 / 61)** | | | | **Day -3 (20 / 48)** | | | |
|---|---|---|---|---|---|---|---|---|---|---|---|---|
| **Number of nucleic acid markers** | **AUC** | **ACC** | **PPV** | **NPV** | **AUC** | **ACC** | **PPV** | **NPV** | **AUC** | **ACC** | **PPV** | **NPV** |
| 16 | 0.908 | 0.848 | 0.823 | 0.867 | 0.927 | 0.876 | 0.860 | 0.891 | 0.887 | 0.856 | 0.817 | 0.889 |
| 15 | 0.908 | 0.842 | 0.804 | 0.865 | 0.915 | 0.858 | 0.839 | 0.879 | 0.881 | 0.857 | 0.828 | 0.887 |
| 14 | 0.895 | 0.829 | 0.763 | 0.861 | 0.911 | 0.855 | 0.831 | 0.880 | 0.885 | 0.853 | 0.825 | 0.881 |
| 13 | 0.894 | 0.826 | 0.759 | 0.860 | 0.913 | 0.860 | 0.835 | 0.885 | 0.891 | 0.847 | 0.815 | 0.876 |
| 12 | 0.900 | 0.831 | 0.768 | 0.863 | 0.924 | 0.873 | 0.883 | 0.881 | 0.865 | 0.794 | 0.737 | 0.827 |
| 11 | 0.879 | 0.818 | 0.729 | 0.864 | 0.908 | 0.851 | 0.835 | 0.870 | 0.872 | 0.807 | 0.779 | 0.832 |
| 10 | 0.865 | 0.814 | 0.720 | 0.862 | 0.912 | 0.852 | 0.833 | 0.872 | 0.875 | 0.815 | 0.774 | 0.843 |
| 9 | 0.865 | 0.816 | 0.723 | 0.861 | 0.913 | 0.856 | 0.839 | 0.875 | 0.876 | 0.807 | 0.770 | 0.836 |
| 8 | 0.871 | 0.813 | 0.731 | 0.856 | 0.915 | 0.852 | 0.818 | 0.878 | 0.866 | 0.796 | 0.743 | 0.829 |
| 7 | 0.876 | 0.811 | 0.734 | 0.850 | 0.903 | 0.839 | 0.824 | 0.857 | 0.861 | 0.808 | 0.752 | 0.836 |
| 6 | 0.867 | 0.807 | 0.728 | 0.849 | 0.887 | 0.803 | 0.748 | 0.840 | 0.858 | 0.818 | 0.801 | 0.836 |
| 5 | 0.850 | 0.797 | 0.720 | 0.837 | 0.868 | 0.819 | 0.778 | 0.851 | 0.793 | 0.787 | 0.721 | 0.826 |
| 4 | 0.836 | 0.773 | 0.657 | 0.818 | 0.867 | 0.807 | 0.739 | 0.851 | 0.785 | 0.779 | 0.698 | 0.828 |
| 3 | 0.834 | 0.778 | 0.675 | 0.820 | 0.868 | 0.808 | 0.761 | 0.836 | 0.794 | 0.808 | 0.763 | 0.842 |
| 2 | 0.815 | 0.757 | 0.658 | 0.798 | 0.815 | 0.801 | 0.721 | 0.842 | 0.755 | 0.747 | 0.643 | 0.799 |

**Table 2. The nucleic acid biomarker sets used in the classification models of Table 1:**

| Number | List of nucleic acids |
|---|---|
| 16 | ICAM2, SLC39A8, FCER1A, NABP1, SLC11A2, HLA-DMB, MIAT, PAFAH2, TEX2, LTA, FBN2, UVSSA, TDRD9, GAS6, SRRM2, TACSTD2 |
| 15 | ICAM2, SLC39A8, FCER1A, NABP1, SLC11A2, HLA-DMB, MIAT, PAFAH2, TEX2, LTA, FBN2, UVSSA, TDRD9, GAS6, SRRM2 |
| 14 | ICAM2, SLC39A8, FCER1A, NABP1, SLC11A2, HLA-DMB, MIAT, PAFAH2, TEX2, LTA, FBN2, UVSSA, TDRD9, GAS6 |
| 13 | ICAM2, SLC39A8, FCER1A, NABP1, SLC11A2, HLA-DMB, MIAT, PAFAH2, TEX2, LTA, FBN2, UVSSA, TDRD9 |
| 12 | ICAM2, SLC39A8, FCER1A, NABP1, SLC11A2, HLA-DMB, MIAT, PAFAH2, TEX2, LTA, FBN2, UVSSA |
| 11 | ICAM2, SLC39A8, FCER1A, NABP1, SLC11A2, HLA-DMB, MIAT, PAFAH2, TEX2, LTA, FBN2 |
| 10 | ICAM2, SLC39A8, FCER1A, NABP1, SLC11A2, HLA-DMB, MIAT, PAFAH2, TEX2, LTA |
| 9 | ICAM2, SLC39A8, FCER1A, NABP1, SLC11A2, HLA-DMB, MIAT, PAFAH2, TEX2 |
| 8 | ICAM2, SLC39A8, FCER1A, NABP1, SLC11A2, HLA-DMB, MIAT, PAFAH2 |
| 7 | ICAM2, SLC39A8, FCER1A, NABP1, SLC11A2, HLA-DMB, MIAT |
| 6 | ICAM2, SLC39A8, FCER1A, NABP1, SLC11A2, HLA-DMB |
| 5 | ICAM2, SLC39A8, FCER1A, NABP1, SLC11A2 |
| 4 | ICAM2, SLC39A8, FCER1A, NABP1 |
| 3 | ICAM2, SLC39A8, FCER1A |
| 2 | ICAM2, SLC39A8 |

Overall 157 nucleic acid markers were identified in the study as a whole as being relevant and highly significant to the prediction of development of infection, or development of organ dysfunction. The 157 nucleic acid markers are:
AC005747.1, AC112777.1, AC132942.1, AC141557.1, ACVR1B, ADCK2, AFDN, AL096803.2, AL133444.1, ANKS1A, ARHGEF40, ARL4A, ASB1, ASPM, ATP5MD, B4GALT5, BAG3, BEX1, BTNL8, C1orf116, C1orf226, C22orf46, C7orf50, CACTIN, CBS, CCDC32, CCNY, CD177P1, CD58, CDCA7, CDH23, CENPE, CGTLC4P, CLASP1, CLECL1, CLN3, CPA3, CPEB4, CXCL16, CXCR2P1, DHRS9, DLGAP5, EIF1AY, EIF4G3, FAM83A, FAR2, FBN2, FBXL18, FBXW9, FCER1A, FCMR, FHDC1, FOCAD, FOXI3, GAS6, GAS7, GGTLC4P, GIMAP5, GRAP2, H2BC11, HBD, HCAR2, HCAR3, HIPK2, HLA-DMB, HNMT, HROB, ICAM2, ID3, IL18RAP, INKA1, IRF5, IRX3, JUP, KCNC4, KIR2DL4, KMT5B, LARP1, LDLR, LDLRAP1, LINC01347, LINC02363, LMNA, LRRN3, LTA, MAD1L1, MBOAT2, MIAT, MRPS6, MSANTD1, MTRR, N4BP2, NABP1, NCAPG2, NCOR2, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SLC51A, SMG1, SNAPC2, SNHG5, SNRPN, SPC24, SPTLC2, SQLE, SRRM2, STAB1, TACSTD2, TCAF1, TDRD9, TEX2, THBS3, THEM6, TK1, TLE1, TMEM116, TMEM144, TMEM183A, TMTC1, TOP2A, TSC22D1, TTC39C, UGP2, UVSSA, VNN1, VPS9D1, ZBTB16, ZNF608, ZNF792.

Of these 157 nucleic acid markers, 30 markers are particularly relevant for predicting infection, as compared to no infection controls:
AC005747.1, ACVR1B, ANKS1A, ARHGEF40, B4GALT5, CBS, CD177P1, CDH23, CGTLC4P, EIF4G3, FBXL18, FOXI3, GAS7, GGTLC4P, HIPK2, IRX3, LDLR, LMNA MIAT, MTRR, NCAPG2, NCOR2, OPLAH, SLC51A, SMG1,SPTLC2, SQLE, THBS3, TLE1, TOP2A, VPS9D1 .

Of these 157 nucleic acid markers, 131 markers are particularly relevant for predicting organ dysfunction, as compared to patients developing infection alone, or all patients not developing organ dysfunction:
AC112777.1, AC132942.1, AC141557.1, ADCK2, AFDN, AL096803.2, AL133444.1, ANKS1A, ARL4A, ASB1, ASPM, ATP5MD, B4GALT5, BAG3, BEX1, BTNL8, C1orf116, C1orf226, C22orf46, C7orf50, CACTIN, CCDC32, CCNY, CD58, CDCA7, CENPE, CLASP1, CLECL1, CLN3, CPA3, CPEB4, CXCL16, CXCR2P1, DHRS9, DLGAP5, EIF1AY, FAM83A, FAR2, FBN2, FBXW9, FCER1A, FCMR, FHDC1, FOCAD, GAS6, GIMAP5, GRAP2, H2BC11, HBD, HCAR2, HCAR3, HLA-DMB, HNMT, HROB, ICAM2, ID3, IL18RAP, INKA1, IRF5, JUP, KCNC4, KIR2DL4, KMTSB, LARP1, LDLRAP1, LINC01347, LINC02363, LRRN3, LTA, MAD1L1, MBOAT2, MIAT, MRPS6, MSANTD1, N4BP2, NABP1, NEAT1, NIPAL3, NTN3, NUSAP1, OAS2, OIP5, OPLAH, PABPC1, PAFAH2, PDXDC1, PFKFB2, PGA4, PGA5, PGD, PID1, PKIA, POLQ, POTEG, PTGS2, RABGEF1, RAP1A, RBMS2P1, RNVU1-7, RWDD2A, SELENOM, SERPINA10, SGSH, SLC11A2, SLC26A8, SLC39A8, SNAPC2, SNHG5, SNRPN, SPC24, SRRM2, STAB1, TACSTD2, TCAF1, TDRD9, TEX2, THEM6, TK1, TMEM116, TMEM144, TMEM183A, TMTC1, TOP2A, TSC22D1, TTC39C, UGP2, UVSSA, VNN1, ZBTB16, ZNF608, ZNF792.

The Applicant has interrogated this nucleic acid marker data, in light of specifically effective subsets for predicting infection or organ dysfunction, and concluded that subsets of 4 or 5 nucleic acid markers (and potentially less) from the 157, 131, 30 should be capable of predicting infection or organ dysfunction with high AUCs, or accuracy, though preferably the subsets of nucleic acid markers are more likely to number at least 7 or 8 nucleic acid markers, though generally the number of nucleic acid markers would be less than 20, and most likely less than 15.

Markers that occur in multiple exemplified subsets are more likely to provide further effective down-selected subsets, and thus the Applicant suggests recurring nucleic acid markers should be combined to provide further subsets for predicting infection and/or organ dysfunction.

### Recurring genes:

Infection - B4GALT5 (2), EIF4G3 (2), FBXL18 (2), GGTLC4P (2), SPTLC2 (2),
Organ Dysfunction - AC112777.1 (4), AC141557.1 (2), AFDN (3), AL096803.2 (2), ASB1 (2), ATP5MD (2), BTNL8 (4), CCNY (2), CDCA7 (7), CLASP1 (2), FBN2 (6), FCER1A (3), FCMR (2), FHDC1 (2), FOCAD (2), GAS6 (2), GRAP2 (4), HLA-DMB (3), HNMT (2), HROB (4), ICAM2 (3), ID3 (3), INKA1 (3), KCNC4 (2), LARP1 (2), LTA (3), MIAT (4), NABP1 (11), NEAT1 (2), NUSAP1 (2), OIP5 (3), PAFAH2 (4), PGA5 (2), PGD (2), POLQ (2), PTGS2 (2), RAP1A (2), RWDD2A (2), SERPINA10 (2), SGSH (2), SLC11A2 (6), SLC26A8 (3), SLC39A8 (13), SNAPC2 (2), SRRM2 (7), TACSTD2 (9), TDRD9 (4), TEX2 (4), TMEM116 (4), TMTC1 (2), UVSSA (4), ZNF792 (2)

Key markers for predicting organ dysfunction include especially SLC39A8, which occurs in 13 down-selected nucleic acid marker signatures, NABP1, which occurs in 11, TACSTD2, which occurs in 9, CDCA7, which occurs in 7, FBN2, which occurs in 6, and SLC11A2, which occurs in 6 down-selected nucleic acid marker signatures. A suitable marker set for organ dysfunction may thus include one or more of these nucleic acids, and could be a signature for example including at least 4 of these nucleic acids (such as SLC39A8, NABP1, CDCA7, FBN2; or SLC39A8, NABP1, CDCA7, TACSTD2; or SLC39A8, NABP1, FBN2, SLC11A2), or perhaps all six of these nucleic acids.

In a further analysis, the Applicant considered organ dysfunction (sepsis) versus all others (control) using the total 1649 nucleic acids differentially expressed (with a fold change of at least 1.3), but irrespective of day before onset of symptoms of sepsis (thus with all days combined in one analysis), and using the Boruta algorithm 5 fold cross validation repeated 25 times. Through undertaking this analysis, 270 gene markers were identified as being particularly relevant to differentiating patients with sepsis from all other individuals. From these 270 potential targets, 47 genes (nucleic acids) were down-selected as the best candidates for differentiating, with an AUC of 0.935 achievable through use of 40 of these nucleic acids.

The 47 genes were TDRD9, SLC39A8, HLA-DMB, DHRS3, PAFAH2, PKIA, NABP1, TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BIN1, HLA-DPA1, SLC26A6, MAFG, GZMK, HVCN1, RPL13A, FBXW2, TCEA3, SLC36A1, RRBP1, LGALS2, ARID5B, CR1, DNAJC5, CPA3, METTL7B, TCTN1, LDHA.

Key markers for the differentiation were again SLC39A8, NABP1, TACSTD2 (most recurring genes throughout the present analysis), in addition to nucleic acids such as PAFAH2, TDRD9, HLA-DMB, PKIA and TEX2. Nucleic acid markers from previous down-selected biomarker sets, such as for differentiating infection from absence of infection and infection plus organ dysfunction (sepsis) from infection alone were also present in the 47, especially markers such as FCER1A, ICAM2, LGALS2, SLC36A1, SLC26A6, HVCN1, SGSH, LDLR, and B4GALT5. The eighteen nucleic acid biomarker signature of PAFAH2, SLC39A8, TDRD9, DHRS3, HLA-DMB, NABP1, PKIA, TEX2, ANKS1A, B4GALT5, BEND2, FCER1A, HLA-DMA, ICAM2, LARP1, LDLR, MIAT, TACSTD2, TRIP13 had an AUC of 0.924 (PPV 0.852) for predicting patients with organ dysfunction, the seven nucleic acid biomarker signature of PAFAH2, SLC39A8, TDRD9, DHRS3, HLA-DMB, NABP1, PKIA, TEX2 had an AUC of 0.904 (PPV 0.800), and the three nucleic acid signature of PAFAH2, SLC39A8, TDRD9 an AUC of 0.864 (PPV 0.720).

This study is the culmination of over 10 years of research into the pathogenesis of sepsis in elective surgery patients. Analysis of the host transcriptome in whole blood samples before and after surgery has led to the identification of a number of key host biomarkers whose expression enables differentiation of sepsis patients from other cohorts. Whilst whole transcriptome studies of sepsis have been widely reported, all have focussed on patients with established symptoms i.e. patients diagnosed with sepsis or infection before sampling began. However, these have limited utility for the early diagnosis of infection, or pre-symptomatic prediction of infection or organ dysfunction. In addition, the complicated nature of whole transcriptomic data has limited its clinical utility for a number of practical and interpretational reasons.

The work reported here is unique since it has sought to address these two issues. Firstly, it has described the early host response that leads to sepsis through characterisation of the transcriptome of patients that go on to develop sepsis. Secondly, it has down-selected genes (nucleic acids) capable of discriminating between infection, organ dysfunction (sepsis) and other patient cohorts, and proved that it is possible to identify clinically useful host biomarker signatures to predict infection and organ dysfunction in advance of symptoms.

In order to understand the host response that leads to sepsis, a unique approach to patient recruitment was adopted. Clarity on the provenance of each patient was considered as important as the clinical data itself for robust modelling of early sepsis pathogenesis. Patients were comparatively well when they entered the study. Apart from the underlying need for surgery, patients were infection free and were in relatively good health. This was underlined by the high rates of uncomplicated recovery observed in the study. However, 3.53% of the patients recruited into this study did develop organ dysfunction (sepsis). The prospective collection of samples before and after surgery enabled the detailed characterisation of changes in gene expression that led to the development of infection or sepsis in this elective surgery cohort. The low incidence of sepsis also gave a large patient cohort from which age/sex/procedure matched patients could be selected for inter-patient comparison. This enabled the effects of age, gender and surgical procedure to be controlled effectively.

Successful down-selection of genes to manageable numbers is vital for transition to a platform. Consequently, a machine learning algorithm approach has been used to select appropriate targets and classify patients based on host gene expression with output compared to clinical diagnosis to determine predictive accuracy. The success of this approach is evidenced by high AUC values and small biomarker signatures (subsets of nucleic acid markers) of between 4 or 5 markers, preferably about 7 or 8 nucleic acid markers, up to about 15 or 16 markers or even more, though preferably lower or less than 20, or lower or less than 15, when comparing infection and comparator, sepsis (organ dysfunction⁺) with infection only patients, and sepsis (organ dysfunction) with all other patients/samples. These biomarker signatures are significantly much smaller than previously reported signatures for pre-symptomatic prediction of infection, and especially for pre-symptomatic prediction of organ dysfunction, and of significantly higher AUCs than previously reported, especially for predicting organ dysfunction (sepsis).

In trying to answer the more difficult question of when an individual will develop sepsis, this study was able to utilise multiple sample time points collected from each patient.

## Claims

1. A method for predicting or monitoring the development of sepsis in a subject, the method comprising determining levels of nucleic acid markers selected from the list consisting of: TDRD9, SLC39A8, HLA-DMB, DHRS3, PAFAH2, PKIA, NABP1, TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BIN1, HLA-DPA1, SLC26A6, MAFG, GZMK, HVCN1, RPL13A, FBXW2, TCEA3, SLC36A1, RRBP1, LGALS2, ARID5B, CR1, DNAJC5, CPA3, METTL7B, TCTN1, and LDHA in a biological sample taken from the subject, wherein the nucleic acid markers at least comprise PAFAH2, SLC39A8, and TDRD9.

2. Method according to Claim 1, wherein the nucleic acid markers at least comprise PAFAH2, SLC39A8, TDRD9, DHRS3, HLA-DMB, NABP1, PKIA, and TEX2.

3. Method according to Claim 1, wherein the nucleic acid markers at least comprise PAFAH2, SLC39A8, TDRD9, DHRS3, HLA-DMB, NABP1, PKIA, TEX2, ANKS1A, B4GALT5, BEND2, FCER1A, HLA-DMA, ICAM2, LARP1, LDLR, MIAT, TACSTD2, and TRIP13.

4. Method according to Claims 1 to 3, wherein the method is computer implemented using an appropriate mathematical model to analyse data generated.

5. A kit for predicting or monitoring development of sepsis in a subject, said kit consisting of reagents and/or systems for specifically determining levels of nucleic acid markers selected from the lists consisting of: TDRD9, SLC39A8, HLA-DMB, DHRS3, PAFAH2, PKIA, NABP1, TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BIN1, HLA-DPA1, SLC26A6, MAFG, GZMK, HVCN1, RPL13A, FBXW2, TCEA3, SLC36A1, RRBP1, LGALS2, ARID5B, CR1, DNAJC5, CPA3, METTL7B, TCTN1, and LDHA in a biological sample taken from the subject, wherein the nucleic acid markers at least comprise PAFAH2, SLC39A8, and TDRD9.

6. A kit according to Claim 5, wherein the reagents and/or systems for specifically determining levels of the nucleic acid markers comprise reagents for nucleic acid amplification, which reagents for nucleic acid amplification comprise one or more of fluorescently-labelled oligonucleotide probes or fluorescently-labelled primers, wherein the fluorescently-labelled oligonucleotide probes or fluorescently-labelled primers consist of probes and primers each capable of specific binding to nucleic acid products of the nucleic acid markers and the nucleic acid amplification is real-time (RT) polymerase chain reaction (PCR).

7. An apparatus for analysis of a biological sample from a subject to predict or monitor the development of sepsis in the subject consisting means for specifically monitoring, measuring or detecting the expression of nucleic acids selected from the list consisting of: TDRD9, SLC39A8, HLA-DMB, DHRS3, PAFAH2, PKIA, NABP1, TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BIN1, HLA-DPA1, SLC26A6, MAFG, GZMK, HVCN1, RPL13A, FBXW2, TCEA3, SLC36A1, RRBP1, LGALS2, ARID5B, CR1, DNAJC5, CPA3, METTL7B, TCTN1, and LDHA in a biological sample from the subject, wherein the nucleic acid markers at least comprise PAFAH2, SLC39A8, and TDRD9; and means for analysis of data produced from the means for specifically monitoring, measuring or detecting, and means for providing an output from the analysis which output provides a prediction of the likelihood of a subject having sepsis, or an output to enable monitoring of sepsis.

8. An apparatus according to Claim 7, wherein the means for analysis of data is computer implemented using an appropriate mathematical model to analyse the data.

## Patentansprüche

1. Verfahren zum Vorhersagen oder Überwachen der Entwicklung einer Sepsis bei einem Patienten, wobei das Verfahren das Bestimmen von Pegeln von Nukleinsäuremarkern umfasst, die aus der Liste bestehend aus TDRD9, SLC39A8, HLA-DMB, DHRS3, PAFAH2, PKIA, NABP1, TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BINI, HLA-DPA1, SLC26A6, MAFG, GZMK, HVCN1, RPL13A, FBXW2, TCEA3, SLC36A1, RRBP1, LGALS2, ARID5B, CRI, DNAJC5, CPA3, METTL7B, TCTN1 und LDHA ausgewählt sind, in einer biologischen Probe, die dem Patienten entnommen wurde, wobei die Nukleinsäuremarker mindestens PAFAH2, SLC39A8 und TDRD9 umfassen.

2. Verfahren nach Anspruch 1, wobei die Nukleinsäuremarker mindestens PAFAH2, SLC39A8, TDRD9, DHRS3, HLA-DMB, NABP1, PKIA und TEX2 umfassen.

3. Verfahren nach Anspruch 1, wobei die Nukleinsäuremarker mindestens PAFAH2, SLC39A8, TDRD9, DHRS3, HLA-DMB, NABP1, PKIA, TEX2, ANKS1A, B4GALT5, BEND2, FCER1A, HLA-DMA, ICAM2, LARP1, LDLR, MIAT, TACSTD2 und TRIP13 umfassen.

4. Verfahren nach den Ansprüchen 1 bis 3, wobei das Verfahren unter Verwendung eines geeigneten mathematischen Modells zur Analyse der generierten Daten computerimplementiert ist.

5. Kit zum Vorhersagen oder Überwachen der Entwicklung einer Sepsis bei einem Patienten, wobei das Kit Reagenzien und/oder Systeme zum spezifischen Bestimmen der Konzentrationen von Nukleinsäuremarkern umfasst, die aus den Listen bestehend aus TDRD9, SLC39A8, HLA-DMB, DHRS3, PAFAH2, PKIA, NABP1, TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BIN1, HLA-DPA1, SLC26A6, MAFG, GZMK, HVCN1, RPL13A, FBXW2, TCEA3, SLC36A1, RRBP1, LGALS2, ARID5B, CRI, DNAJC5, CPA3, METTL7B, TCTN1 und LDHA ausgewählt sind, in einer biologischen Probe, die dem Patienten entnommen wurde, wobei die Nukleinsäuremarker mindestens PAFAH2, SLC39A8 und TDRD9 umfassen.

6. Kit nach Anspruch 5, wobei die Reagenzien und/oder Systeme zur spezifischen Bestimmung der Konzentrationen der Nukleinsäuremarker Reagenzien zur Nukleinsäureamplifikation umfassen, wobei die Reagenzien zur Nukleinsäureamplifikation eine oder mehrere fluoreszenzmarkierte Oligonukleotid-Sonden oder fluoreszenzmarkierte Primer umfassen, wobei die fluoreszenzmarkierten Oligonukleotid-Sonden oder fluoreszenzmarkierten Primer aus Sonden und Primern bestehen, die jeweils in der Lage sind, spezifisch an Nukleinsäureprodukte der Nukleinsäuremarker zu binden, und die Nukleinsäureamplifikation eine Echtzeit-Polymerasekettenreaktion (RT-PCR) ist.

7. Vorrichtung zur Analyse einer biologischen Probe von einem Patienten, um die Entwicklung einer Sepsis bei dem Patienten vorherzusagen oder zu überwachen, bestehend aus Mitteln zur spezifischen Überwachung, Messung oder Detektion der Expression von Nukleinsäuren, die aus der Liste bestehend aus TDRD9, SLC39A8, HLA-DMB, DHRS3, PAFAH2, PKIA, NABP1,TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BIN1, HLA-DPA1, SLC26A6, MAFG, GZMK, 15 HVCN1, RPL13A, FBXW2, TCEA3, SLC36A1, RRBP1, LGALS2, ARID5B, CRI, DNAJC5, CPA3, METTL7B, TCTN1 und LDHA ausgewählt sind, in einer biologischen Probe des Patienten, wobei die Nukleinsäuremarker mindestens PAFAH2, SLC39A8 und TDRD9 umfassen; und Mitteln zur Analyse der von den Mitteln zur spezifischen Überwachung, Messung oder Detektion erzeugten Daten und Mitteln zur Bereitstellung einer Ausgabe aus der Analyse, wobei die Ausgabe eine Vorhersage der Wahrscheinlichkeit einer Sepsis bei einem Patienten oder eine Ausgabe zur Ermöglichung der Überwachung einer Sepsis bereitstellt.

8. Vorrichtung nach Anspruch 7, wobei die Mittel zur Analyse von Daten computerimplementiert sind und ein geeignetes mathematisches Modell zur Analyse der Daten verwenden.

## Revendications

1. Procédé pour prédire ou surveiller le développement d'une septicémie chez un sujet, le procédé comprenant la détermination de niveaux de marqueurs d'acide nucléique sélectionnés parmi la liste constituée de : TDRD9, SLC39A8, HLA-DMB, DHRS3, PAFAH2, PKIA, NABP1, TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BIN1, HLA-DPA1, SLC26A6, MAFG, GZMK, HVCN1, RPL13A, FBXW2, TCEA3, SLC36A1, RRBP1, LGALS2, ARID5B, CR1, DNAJC5, CPA3, METTL7B, TCTN1, et LDHA dans un échantillon biologique prélevé sur le sujet, dans lequel les marqueurs d'acide nucléique comprennent au moins PAFAH2, SLC39A8, et TDRD9.

2. Procédé selon la revendication 1, dans lequel les marqueurs d'acide nucléique comprennent au moins PAFAH2, SLC39A8, TDRD9, DHRS3, HLA-DMB, NABP1, PKIA et TEX2.

3. Procédé selon la revendication 1, dans lequel les marqueurs d'acide nucléique comprennent au moins PAFAH2, SLC39A8, TDRD9, DHRS3, HLA-DMB, NABP1, PKIA, TEX2, ANKS1A, B4GALT5, BEND2, FCER1A, HLA-DMA, ICAM2, LARP1, LDLR, MIAT, TACSTD2 et TRIP13.

4. Procédé selon les revendications 1 à 3, dans lequel le procédé est mis en œuvre par ordinateur à l'aide d'un modèle mathématique approprié pour analyser les données générées.

5. Kit pour prédire ou surveiller le développement d'une septicémie chez un sujet, ledit kit étant constitué de réactifs et/ou systèmes permettant de déterminer spécifiquement des niveaux de marqueurs d'acide nucléique sélectionnés parmi les listes constituées de: TDRD9, SLC39A8, HLA-DMB, DHRS3, PAFAH2, PKIA, NABP1, TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BIN1, HLA-DPA1, SLC26A6, MAFG, GZMK, HVCN1, RPL13A, FBXW2, TCEA3, SLC36A1, RRBP1, LGALS2, ARID5B, CR1, DNAJC5, CPA3, METTL7B, TCTN1, et LDHA dans un échantillon biologique prélevé sur le sujet, dans lequel les marqueurs d'acide nucléique comprennent au moins PAFAH2, SLC39A8 et TDRD9.

6. Kit selon la revendication 5, dans lequel les réactifs et/ou systèmes pour déterminer spécifiquement des niveaux des marqueurs d'acide nucléique comprennent des réactifs pour l'amplification d'acide nucléique, lesquels réactifs pour l'amplification d'acide nucléique comprennent une ou plusieurs sondes oligonucléotidiques marquées par fluorescence ou amorces marquées par fluorescence, dans lequel les sondes oligonucléotidiques marquées par fluorescence ou amorces marquées par fluorescence consistent en des sondes et des amorces chacune capable de se lier spécifiquement aux produits d'acide nucléique des marqueurs d'acide nucléique et l'amplification d'acide nucléique est une réaction en chaîne par polymérase (PCR) en temps réel (RT).

7. Appareil pour analyser un échantillon biologique provenant d'un sujet afin de prédire ou surveiller le développement d'une septicémie chez le sujet, consistant en un moyen pour spécifiquement surveiller, mesurer ou détecter l'expression d'acides nucléiques sélectionnés dans la liste constituée de : TDRD9, SLC39A8, HLA-DMB, DHRS3, PAFAH2, PKIA, NABP1, TEX2, FCER1A, TACSTD2, BEND2, MIAT, ICAM2, LARP1, ANKS1A, HLA-DMA, TRIP13, B4GALT5, SGSH, NDST2, LDLR, HLA-DPB1, SLC2A11, EIF4G3, QSOX1, BIRC5, CD3D, ATP2A2, BIN1, HLA-DPA1, SLC26A6, MAFG, GZMK, HVCN1, RPL13A, FBXW2, TCEA3, SLC36A1, RRBP1, LGALS2, ARID5B, CR1, DNAJC5, CPA3, METTL7B, TCTN1, et LDHA dans un échantillon biologique prélevé sur le sujet, dans lequel les marqueurs d'acide nucléique comprennent au moins PAFAH2, SLC39A8 et TDRD9 ; et un moyen pour analyser des données produites à partir des moyens pour spécifiquement surveiller, mesurer ou détecter, et un moyen pour fournir une sortie à partir de l'analyse, laquelle sortie fournit une prédiction de la probabilité qu'un sujet souffre de septicémie, ou une sortie permettant de surveiller la septicémie.

8. Appareil selon la revendication 7, dans lequel le moyen pour analyser des données est mis en œuvre par ordinateur à l'aide d'un modèle mathématique approprié pour analyser les données.
